# FASCICULE DE BREVET EUROPEEN

(11) **EP 0 995 121 B1**
(45) Date de publication et mention de la délivrance du brevet: **26.05.2004**
(21) Numéro de dépôt: 98932208.6
(22) Date de dépôt: 15.06.1998
(51) Int. Cl.: G01N 33/74, G01N 33/68, G01N 33/577, C07K 16/26, C12N 5/18

(54) **PROCEDE DE DOSAGE SPECIFIQUE DU C-PEPTIDE**
VERFAHREN ZUR SPECIFISCHEN BESTIMMUNG DER C-PEPTIDE
C-PEPTIDE SPECIFIC ASSAY PROCEDURE

(30) Priorité: 20.06.1997 FR 9707730
(43) Date de publication de la demande: 26.04.2000
(73) Titulaire: PASTEUR SANOFI DIAGNOSTICS, 92430 Marnes La Coquette (FR)
(72) Inventeur: BOUANANI, Majida, F-34000 Montpellier (FR); KAMAL, Nadia, F-34060 Montpellier (FR); LARUE, Catherine, Christiane, Marie, F-92420 Vaucresson (FR); MANI, Jean-Claude, F-34000 Montpellier (FR); PAU, Bernard, Christian, F-34000 Montpellier (FR); SIOHAN, Elisabeth, F-69100 Villeurbanne (FR)
(74) Mandataire: Bernasconi, Jean Raymond
(86) Numéro de dépôt international: PCT/FR1998/001253
(87) Numéro de publication internationale: WO 1998/059246

(56) Documents cités:
- EP-A- 0 484 961
- US-A- 4 722 889
- MADSEN ET AL.: "The production and characterization of monoclonal antibodies specific for human proinsulin using a sensitive microdot assay procedure" ENDOCRINOLOGY, vol. 113, no. 6, 1983, pages 2135-2144, XP002058659
- ANGELO ET AL.: "A highly sensitive monoclonal antibody against human C-peptide" DIABETES RESEARCH AND CLINICAL PRACTICE, vol. suppl. 1, 1985, AMSTERDAM, pages S18-S19, XP002058660 cité dans la demande
- REAVEN ET AL.: "Plasma insulin, C-peptide, and proinsulin concentrations in obese and nonobese individuals with varying degrees of glucose tolerance" JOURNAL OF CLINICAL ENDOCRINOLOGY AND METABOLISM, vol. 76, no. 1, 1993, pages 44-48, XP002058661 cité dans la demande

## Description

La présente invention concerne un procédé de dosage spécifique du C-peptide, qui permet d'éliminer toute interférence due à la proinsuline et à ses intermédiaires, notamment à la des-31,32-proinsuline et/ou à la des-64,65-pro-insuline. Elle concerne également des anticorps qui permettent de mettre en oeuvre ce dosage.

L'insuline est synthétisée par les cellules β des îlots de Langerhans à partir d'un précurseur, la proinsuline. La proinsuline subit l'action d'enzymes protéolytiques qui en détachent l'insuline (masse moléculaire 5.800 Daltons) et le C-peptide (masse moléculaire 3.020 Daltons). Cette hydrolyse. met en jeu deux types de protéases, l'une de type carboxypeptidase et l'autre de type endopeptidase. Le processus engendre des composés intermédiaires tels que la split-32,33-proinsuline, la split-65,66-proinsuline, la des-31,32-proinsuline, et la des-64,65-proinsuline, qui avec la proinsuline intacte subsistent en faibles proportions (2 à 6 %) aux côtés de l'insuline et du C-peptide.

Le rapport équimolaire d'insuline et de C-peptide au niveau portal n'est pas retrouvé au niveau périphérique. Dans le sang veineux périphérique à l'état basal, il y a en moyenne 3 à 6 fois plus de C-peptide que d'insuline. Ce phénomène s'explique par les différences de métabolisme entre ces deux polypeptides. Près de 50 % de l'insuline est inactivée au cours de la traversée hépatique. Les quantités restantes sont inactivées au niveau de ses récepteurs périphériques présents notamment dans les tissus musculaires ou adipeux. Par contre, le C-peptide traverse presque librement le foie et, de ce fait, a une demi-vie plasmatique beaucoup plus longue que l'insuline (30 minutes pour le C-peptide et inférieure à 5 minutes pour l'insuline). Le C-peptide ne possède aucune action biologique connue. Son rôle se limite au repliement de la molécule de proinsuline dans les cellules β du pancréas. il apparaît dans le secteur vasculaire comme un simple résidu, avant d'être éliminé sous forme intacte par le rein.

Le dosage plasmatique ou urinaire du C-peptide constitue donc un moyen d'évaluation de l'insulinosécrétion beaucoup plus fiable que le dosage d'insuline. A noter que le dosage du C-peptide permet une estimation de la production d'insuline endogène, même lors de l'administration chez le patient d'insuline exogène ou en présence d'anticorps anti-insuline qui ne permettent pas le dosage immunologique direct de l'insuline.

Le dosage du C-peptide trouve en particulier son application dans le diagnostic différentiel entre le diabète de type 1 et le diabète de type Il, l'évaluation de la fonction pancréatique résiduelle des cellules β, la détection et la surveillance de la phase de rémission du diabète de type I, le dépistage des hypoglycémies factices provoquées par injection d'insuline, dans le diagnostic de l'insulinome et l'évaluation de la sécrétion d'insuline au cours des affections hépatiques.

Le dosage du C-peptide est également utile pour établir un pronostic de diabète pour le foetus lors de la surveillance de la grossesse chez les femmes diabétiques. Par ailleurs, la mesure du C-peptide est utilisée lors de la surveillance de la pancréatectomie totale.

Des procédés de dosages du C-peptide sont connus. La demande de brevet EP 227351 décrit l'utilisation d'un antisérum anti-C-peptide pour mesurer le C-peptide dans un dosage radioimmunologique par compétition. Des anticorps monoclonaux anti-C-peptide utiles dans des tests radioimmunologiques sont également décrits par Angelo et al (*Diabetes Research and Clinical Practice (1985) vol. suppl. 1, p. 18-19*). Comme dans le cas précédent, ces anticorps trouvent leur application dans un immunodosage par compétition. Par ailleurs, Madsen et al. (Endocrinology (1983) 113 n° 6 pp. 2135-2144) ont décrit la production et la caractérisation d'anticorps monoclonaux spécifiques de la proinsuline humaine. La demande EP 484961 couvre un procédé de détection et de dosage de type "sandwich" du C-peptide. Selon cette demande, ce procédé peut être réalisé en utilisant des anticorps monoclonaux ou polyclonaux et l'évaluation du C-peptide peut être notamment réalisée par la technique ELISA, par exemple.

Le brevet CS 277597, publié le 17 mars 1993, décrit également des anticorps monoclonaux, utilisés pour la détermination de la proinsuline ou du C-peptide.

Il est également connu que la quasi-totalité des anticorps monoclonaux ou polyclonaux anti- C-peptide se lient plus ou moins de manière croisée avec la proinsuline et les formes intermédiaires produites avant le clivage en insuline et C-peptide. En effet, les anticorps polyclonaux ou monoclonaux, produits à partir du C-peptide seul sont capables de reconnaître. le C-peptide dans la proinsuline et dans les formes intermédiaires produites avant le clivage en insuline et C-peptide, du fait de la grande homologie entre C-peptide libre et C-peptide lié à l'insuline qui forme la proinsuline.

Chez le sujet normal, les concentrations de C-peptide sont environ 50 fois plus élevées que celles des proinsulines (environ 0.5-1.5 nM contre 5-25 pM à jeun). Cependant, dans certains cas de DNID (diabète non-insulinodépendant), chez les diabétiques de type I nouvellement découverts ainsi que dans leur fratrie, au cours de l'insulinôme et dans les hyperproinsulinémies familiales, les concentrations plasmatiques des proinsulines sont augmentées et peuvent dépasser 2000 pM. Dans ces situations, les principales formes présentes dans la circulation sanguine sont la proinsuline intacte et la des-31,32-proinsuline. Les autres intermédiaires, ne sont présents dans la circulation sanguine qu'à des quantités infimes (Reaven et al, *J. Clin. Endocrinol. Metabol.* (1993), 1, pp. 44-48).

Il est donc important de mettre en oeuvre des procédés d'évaluation spécifiques du C-peptide, qui permettent d'éliminer les interférences dues à la proinsuline intacte et à la des-31,32-proinsuline afin de rendre un résultat fiable dans toute situation physiopathologique. Dinesens et al *("First assay specific for intact human proinsuline", communication réalisée à EASD'94 30th annual meeting - Düsseldorf 27 septembre - 1 octobre 1994)* ont proposé un dosage spécifique de la proinsuline - sans interférence due à l'insuline - en utilisant deux anticorps spécifiques des jonctions AC (jonction entre la chaîne A de l'insuline et le C-peptide) et BC (jonction entre la chaîne B de l'insuline et le C-peptide) de la proinsuline. Toutefois, ces auteurs n'ont pas proposé un dosage spécifique du C-peptide permettant d'éliminer les interférences dues aux proinsulines.

L'utilisation d'anticorps spécifiques pour éliminer des interférences dues à la présence des substances biologiques apparentées (« anticorps de masquage ») est déjà décrit dans l'art antérieur. Par exemple, le brevet US 4 722 889 décrit un procédé de dosage spécifique de la gonadotrophine chorionique humaine (hCG) de type sandwich utilisant deux anticorps capables de reconnaître également la thyréostimuline (TSH), la folliculostimuline (FSH) et la lutéostimuline (LH). Pour éliminer les interférences dues à ces trois dernières hormones, un troisième anticorps de masquage qui reconnaît spécifiquement l'épitope de l'unité β de la- TSH, de la FSH et de la LH et qui a une affinité moindre pour l'unité β de Ia hCG est utilisé. De plus, quand l'antigène interférent est en excès, il y a saturation d'anticorps non spécifique de la phase solide et donc sous-évaluation de l'anticorps à doser. Par contre, il n'a jamais été décrit dans l'art antérieur que pour obtenir un dosage spécifique, il est possible d'utiliser un, anticorps qui reconnaît la substance à doser ainsi que le précurseur de cette substance considéré comme interférent. Dans ce cas, il est possible d'envisager tous les types de dosage, parmi lesquels des dosages de type compétitif

La Demanderesse a maintenant trouvé un procédé de dosage spécifique du C-peptide, qui permet d'éliminer toute interférence due à la des-31,32-proinsuline et/ou à la des-64,65-proinsuline. En effet, grâce à l'utilisation d'anticorps spécifiques de la proinsuline intacte, de la des-31,32-proinsuline et/ou de la des-64,65-proinsuline, reconnaissant un épitope adjacent à l'épitope de l'anticorps anti-C-peptide, il est possible de procéder au dosage du C-peptide dans les milieux biologiques avec une grande spécificité et une grande précision.

L'invention concerne un procédé de dosage du C-peptide dans lequel un échantillon susceptible de contenir le C-peptide est mis en contact avec un ou plusieurs anticorps anti-C-peptide et
- soit un anticorps anti-proinsuline spécifique de la proinsuline intacte et de la des-31,32-proinsuline, reconnaissant un épitope soit adjacent, soit chevauchant, à l'épitope de l'anticorps anti-C-peptide gênant ainsi la fixation de l'anticorps anti-C-peptide sur la proinsuline intacte et la des-31,32-proinsuline,
- soit un anticorps anti-proinsuline spécifique de la proinsuline intacte et de la des-64,65-proinsuline, reconnaissant un épitope soit adjacent, soit chevauchant, à l'épitope de l'anticorps anti-C-peptide gênant ainsi la fixation de l'anticorps anti-C-peptide sur la proinsuline intacte et la des-64,65-proinsuline,
- soit un mélange dudit anticorps anti-proinsuline spécifique de la proinsuline intacte et de la des-31,32-proinsuline et dudit anticorps anti proinsuline spécifique de la proinsuline intacte et de la des-64,65-proinsuline.

Selon un mode de réalisation particulier de l'invention, un échantillon susceptible de contenir le C-peptide est mis en contact :
- soit avec un anticorps anti-C-peptide et
   - un anticorps anti-proinsuline spécifique de la proinsuline intacte et de la des-31,32-proinsuline, reconnaissant un épitope soit adjacent, soit chevauchant, à l'épitope de l'anticorps anti-C-peptide gênant ainsi la fixation de l'anticorps anti-C-peptide sur la proinsuline intacte et la des-31,32-proinsuline,
   - ou un anticorps anti-proinsuline spécifique de la proinsuline intacte et de la des-64,65-proinsuline, reconnaissant un épitope soit adjacent, soit chevauchant, à l'épitope de l'anticorps. anti-C-peptide gênant ainsi la fixation de l'anticorps anti-C-peptide sur la proinsuline intacte et la- des-64,65-proinsuline,
- soit avec un mélange d'un premier anticorps anti-C-peptide, d'un anticorps anti-proinsuline spécifique de la proinsuline intacte et de la des-31,32-proinsuline, reconnaissant un épitope soit adjacent, soit chevauchant, à l'épitope dudit premier anticorps anti-C-peptide gênant ainsi la fixation dudit premier anticorps anti-C-peptide sur la proinsuline intacte et la des-31,32-proinsuline,
et d'un deuxième anticorps anti-C-peptide, d'un anticorps anti-proinsuline spécifique de la proinsuline intacte et de la des-64,65-proinsuline, reconnaissant un épitope adjacent, soit chevauchant à l'épitope- dudit deuxième anticorps anti-C-peptide gênant ainsi la fixation dudit deuxième anticorps anti-C-peptide sur la proinsuline intacte et la des-64,65-proinsuline,
ledit premier et ledit deuxième anticorps anti-C-peptide étant éventuellement identiques.

Dans le cas où ledit premier et ledit deuxième anticorps anti-C-peptide sont identiques, un échantillon susceptible de contenir le C-peptide est donc mis en contact avec un anticorps anti-C-peptide, un anticorps anti-proinsuline spécifique de la proinsuline intacte et de la des-31,32-proinsuline, et un anticorps anti-proinsuline spécifique de la proinsuline intacte et de la des-64,65-proinsuline.

Selon un mode préféré de réalisation de l'invention, ledit épitope reconnu par l'anticorps spécifique de la proinsuline intacte et de la des-31,32-proinsuline, est situé dans la jonction AC de la proinsuline et de la des-31,32-proinsuline et inclut la séquence peptidique choisie parmi:
la séquence LysArg,
de préférence la séquence Leu Gln Lys Arg Gly lle Val Glu
et de préférence encore la séquence Leu Gln Lys Arg Gly Ile Va) Glu Gln.

De manière préférentielle, ledit anticorps anti-proinsuline selon l'invention inhibe d'au moins 50 % la liaison de l'anticorps anti-C-peptide à la proinsuline lorsqu'il est présent à la même concentration que l'anticorps anti-C peptide.

Selon un autre mode préféré de l'invention, ledit épitope reconnu par l'anticorps spécifique de la proinsuline intacte et la des-64,65-proinsuline, est situé dans la jonction BC de la proinsuline et la des-64,65-proinsuline et inclut la séquence peptidique :

Arg Arg.

Les anticorps utilisés pour la mise en oeuvre du procédé, selon l'invention, peuvent être des anticorps monoclonaux ou des anticorps polyclonaux. On préfère mettre en oeuvre au moins un anticorps monoclonal.

Comme anticorps spécifiques de la des-31,32-proinsuline et de la proinsuline, on peut utiliser en particulier, des anticorps monoclonaux qui reconnaissent un épitope soit adjacent, soit chevauchant, à l'épitope de l'anticorps anti-C-peptide, gênant ainsi la fixation de l'anticorps anti-C-peptide sur la proinsuline intacte et la des-31,32-proinsuline, ledit épitope étant situé dans la jonction AC de la proinsuline et la des-31,32-proinsuline et la séquence incluant une séquence peptidique choisie parmi :

Lys Arg,

préférentiellement la séquence:

Leu Gln Lys Arg Gly lle Val Glu

et plus spécialement la séquence :

Leu Gln Lys Arg Gly lle Val Glu Gln.

Un tel anticorps peut être notamment l'anticorps monoclonal AC1D4, obtenu à partir d'un hybridome déposé auprès de la Collection Nationale de Cultures de Microorganismes de l'Institut Pasteur, le 4 juin 1997, sous le numéro I-1873.

Pour mettre en oeuvre le procédé selon l'invention on peut utiliser des anticorps spécifiques de la des-64,65-proinsuline et de la proinsuline qui reconnaissent un épitope soit adjacent, soit chevauchant à l'épitope de l'anticorps anti-C-peptide, gênant ainsi la fixation de l'anticorps anti-C-peptide sur la proinsuline intacte et la des-64,65-proinsuline, ledit épitope étant situé dans la jonction BC de la proinsuline et la des-64,65-proinsuline et incluant la séquence peptidique choisie parmi:
la séquence Arg Arg,
la séquence Phe Tyr Thr Pro Lys Thr Arg Arg Glu Ala Glu Asp: Leu Gln Val,
la séquence Thr Pro Lys Thr Arg Arg Glu Ala Glu Asp Leu Gln Val,
et la séquence Phe Tyr Thr Pro Lys Thr Arg Arg Glu Ala Glu Asp Leu.

On préfère les anticorps spécifiques de la des-64,65-proinsuline et de la proinsuline, qui reconnaissent un épitope correspondant aux séquences :

Thr Pro Lys Thr Arg Arg Glu Ala Glu Asp Leu Gln Val

ou

Phe Tyr Thr Pro Lys Thr Arg Arg Glu Ala Glu Asp Leu.

Il est évident que lorsqu'on utilise un anticorps spécifique de la proinsuline intacte et de la des-31,32-proinsuline, il est possible d'éviter les interférences dues à la liaison de l'anticorps anti-C-peptide avec la split-32,33-proinsuline. De même, en utilisant un anticorps spécifique de la proinsuline intacte et de la des-64,65-proinsuline, il est possible d'éviter les interférences dues à la liaison de l'anticorps anti-C-peptide avec la split-65,66-proinsuline.

Le procédé selon l'invention est utile pour le dosage du C-peptide dans des échantillons biologiques tels que plasma, sérum ou urines.

Un procédé de dosage préféré selon l'invention est un dosage du type « sandwich ». Selon ce type de dosage, un anticorps anti-C-peptide, monoclonal ou polyclonal, peut être fixé sur une phase solide. On préfère que cet anticorps anti-C-peptide soit un anticorps monoclonal. Après la mise en contact avec l'échantillon biologique à doser, un deuxième anticorps anti-C-peptide lié à un marqueur, par exemple enzymatique, est ajouté. Les anticorps qui permettent, selon l'invention, le dosage spécifique du C-peptide (notamment l'anticorps spécifique de la des-31,32-proinsuline et éventuellement l'anticorps spécifique de la des-64,65-proinsuline) peuvent être mis en oeuvre, soit simultanément avec le deuxième anticorps anti-C-peptide, soit avant ce dernier. Ce dosage peut être réalisé soit en un temps, soit en deux temps et l'évaluation du C-peptide peut être effectuée en utilisant toute méthode de détection, telle que la détection enzymatique, ou la détection utilisant un agent radioactif, fluorescent, chimioluminescent, etc.

Un autre type de dosage, qui peut être mis en oeuvre selon le procédé de l'invention, est un dosage radioimmunologique de type « compétitif ». Selon ce type de dosage, on met en contact un anticorps anti-C-peptide, monoclonal ou polyclonal, qui peut être fixé sur une phase solide, avec un échantillon biologique à doser. Puis, on introduit l'anticorps spécifique de la des-31,32-proinsuline, et/ou l'anticorps spécifique de la des-64,65-proinsuline, ainsi que du C-peptide marqué par un marqueur détectable tel qu'un agent cité ci-dessus.

Les trousses qui contiennent un anticorps anti-C-peptide et un anticorps spécifique de la des-31,32-proinsuline et de la proinsuline et/ou un anticorps spécifique de la des-64-,65-proinsuline et de la proinsuline, font également partie de la présente invention. Ces trousses permettent de mettre en oeuvre le procédé de dosage spécifique du C-peptide. Les trousses, selon l'invention, sont utilisées notamment pour la mise en oeuvre d'un dosage, soit de type sandwich, soit de type compétitif, spécifique du C-peptide.

### PRODUCTION ET CARACTERISATION DE L'ANTICORPS MONOCLONAL AC1D4:

### 1. Production

L'anticorps monoclonal AC1D4 a été obtenu par hybridation lymphocytaire.
Des souris Balb/c femelles de 6 semaines ont reçu au total 4 injections intrapéritonéales de proinsuline humaine. Au jour 1 les souris ont été immunisées avec 50 µg de proinsuline humaine par voie intrapéritonéale en présence d'adjuvant complet de Freund (ACF). Trois semaines plus tard les souris ont été immunisées de nouveau avec 50 µg de proinsuline humaine en présence d'adjuvant incomplet de Freund. Une troisième immunisation a été réalisée 3 semaines plus tard, dans les mêmes conditions que précédemment en utilisant une solution de la proinsuline humaine dans une solution saline de tampon phosphate à pH 7,4 (PBS). Des échantillons de sang de souris ont été prélevés après la deuxième et la troisième injections, 12 jours après chaque injection. Les sérums ont été testés en ELISA, RIA et en SPOT *(Franck R. Tetrahedron,* (*1992), 48*, *p 9217-9232)* pour la présence. d'anticorps anti-proinsuline humaine.

Les souris ayant un fort taux sérique d'anticorps anti-proinsuline ont été choisies pour une fusion lymphocytaire. Les souris sélectionnées ont reçu une dernière injection de proinsuline un mois après la troisième injection. La dose de 20 µg a été injectée en deux fois. Trois jours plus tard les souris ont été décapitées et leur rate prélevée. Après broyage de la rate, les lymphocytes ont été fusionnés avec les cellules myélomateuses de la souris de la lignée P3-X63-Ag8.653 en présence de polyéthylèneglycol. La technique d'hybridation lymphocytaire qui a été utilisée est celle de ClonaCell™ proposée par StemCell technologies Inc (distribué en France par TEBU, Le Perray Yvelines). La. sélection des hybridomes sécréteurs d'anticorps a été assurée par une série d'essais menés en parallèle en ELISA (utilisation de la proinsuline humaine immobilisée) et RIA (utilisation de la proinsuline humaine iodée). Les hybridomes sécréteurs ont été analysés en SPOT pour leur activité anti-jonctions AC ou BC.

Pour la mise en oeuvre de la méthode ELISA, des microplaques NUNC ont été revêtues avec de la proinsuline humaine, en utilisant une solution de proinsuline humaine (C= 0,1 µg/ml ) dans du PBS, sous un volume de 100 µl. Les plaques ont été incubées pendant une nuit à +4°C. Après trois lavages avec une solution saline de tampon phosphate additionnée de Tween 20 à 0,1% (PBS-Tween), 200 µl de surnageant de culture ou 100 µl d'anticorps purifiés en concentrations décroissantes ont été déposés dans les microplaques et incubés pendant 3 heures à température ambiante: Ensuite, les microplaques ont été lavées trois fois avec du PBS-Tween et additionnées d'un deuxième anticorps. Cet anticorps, dirigé contre les IgG de souris (spécificité gamma), a été couplé à la peroxydase et a été mis en solution dans PBS-Tween (Dilution 1/3000). Les microplaques ont été incubées pendant une heure à 37°C, puis lavées trois fois avec du PBS-Tw. La révélation de la réaction a été effectuée en utilisant comme substrat l'orthophényldiamine en solution (C = 30 mg/ml) dans une solution de tampon citrate pH=5 contenant 0,03% de peroxyde d'hydrogène. Après 20 minutes d'incubation à température ambiante et à l'abri de la lumière, l'absorbance a été lue à λ=450 nm. La réaction enzymatique peut être arrêtée par 50 µl d'acide sulfurique 4N, la lecture se fait alors à λ=490 nm.

Pour la mise en oeuvre de la méthode RIA, 100 µl de surnageant de culture ou 100 µl d'anticorps purifiés en concentrations décroissantes ont été déposés dans les tubes revêtus avec des anticorps de mouton anti-IgG de souris. Après 3 heures d'incubation à température ambiante, les tubes ont été lavés trois fois avec 2 ml de PBS, puis y sont ajoutés 100 µl de proinsuline. iodée à 3000 cpm en solution dans du PBS contenant 1% de BSA. Les tubes sont incubés pendant une nuit à température ambiante, puis lavés trois fois au PBS (2 ml/tube) et ensuite sont introduits dans un compteur gamma pour lecture.

Un des hybridomes ainsi retenus a été le AC1D4. Cet hybridome a été cloné par dilution limite (*Lefkovits I and Waldmann S. 1979, Cambridge University Press, Cambridge*). Selon cette technique, la suspension cellulaire est diluée de 10 en 10 jusqu'à l'obtention d'une solution à 5 cellules/ml. Cette solution est répartie dans les microplaques de culture cellulaire à raison de 100 µl par puits. Ultérieurement, l'examen au microscope inversé permet de contrôler le développement cellulaire et de retenir les puits présentant un seul clone.

### 2. Caractérisation

### 2.1. Spécificité antigénique

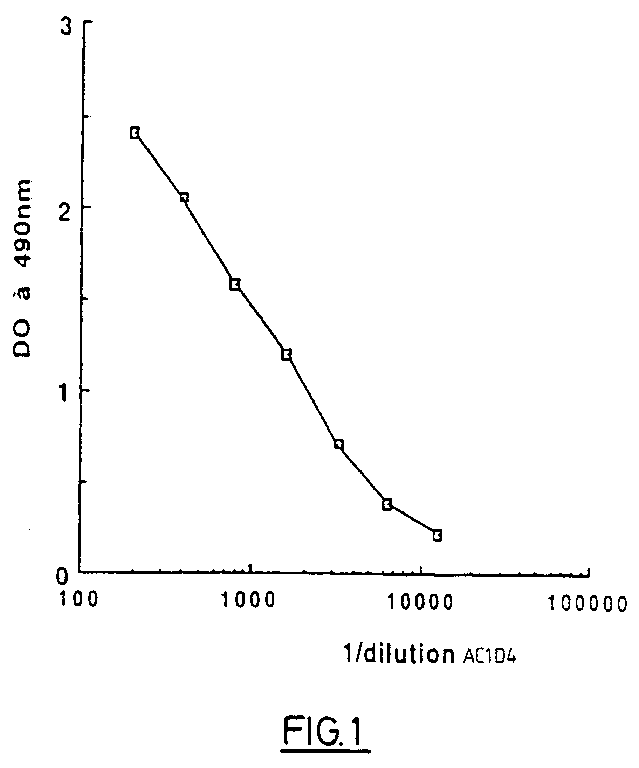

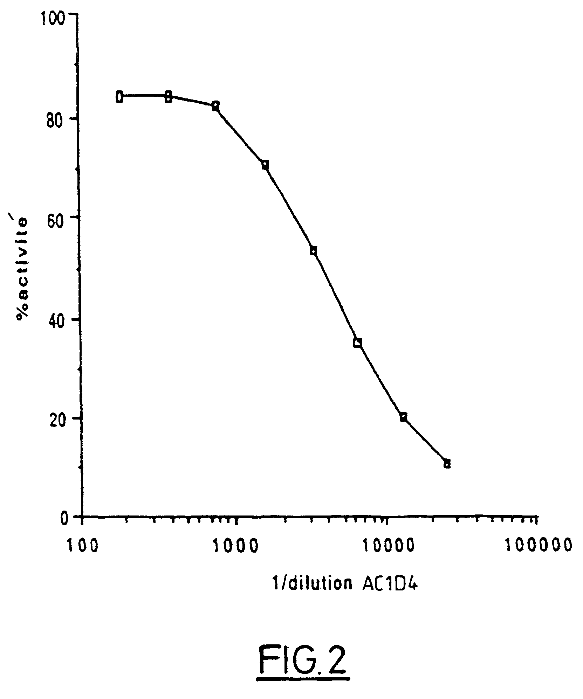

L'anticorps AC1D4 d'isotype IgG1 reconnaît :
- la proinsuline humaine et la des-31,32-proinsuline dans le format ELISA (proinsuline immobilisée)
- la proinsuline humaine et la des-31,32-proinsuline dans le format RIA (proinsuline iodée en solution).
Les résultats de ces études sont donnés dans les figures 1 et 2.

La figure 1 représente la liaison de l'anticorps AC1D4 à la proinsuline en ELISA.

La figure 2 représente la liaison de l'anticorps AC1D4 à la proinsuline marquée en RIA.

### 2.2. Réactions croisées

Des essais de compétition par l'insuline et le C-peptide humain de la liaison de l'anticorps AC1D4 à la proinsuline humaine iodée ont été réalisés par RIA. Les conditions expérimentales ont été identiques à celles de la méthode RIA indiquées précédemment (voir Production) à l'exception de l'étape d'incubation de la proinsuline. Dans le cas présent, après l'étape de l'incubation de l'anticorps AC1D4 dans les tubes (qui est suivie de lavages), l'insuline ou le C-peptide, à 10 µg/ml, ont été incubés en même temps que la proinsuline iodée.

Les résultats ont montré que l'insuline humaine et le C-peptide n'inhibent pas la liaison de l'anticorps AC1D4 à la proinsuline iodée.

### 2.3. Détermination de la séquence de l'épitope

La séquence de l'épitope a été déterminée par la technique SPOT™. Sur une membrane de nitrocellulose, des peptides constitués de 9 acides aminés, et chevauchants entre eux de 8 acides aminés, sont synthétisés. Les peptides synthétisés recouvrent ainsi la totalité de la molécule de la proinsuline humaine (soit au total 78 peptides). Cette membrane a servi de support antigénique pour analyser la séquence reconnue par l'anticorps monoclonal à tester.

Le protocole utilisé, pour la réaction immunologique après synthèse peptidique, est le suivant :
La membrane a été saturée dans un tampon de saturation contenant 10% de CRB (Cambridge Research Biochemicals commercialisé par GENOSYS ref. : SU-07-250) 0,5% de Tween et 5% de saccharose dans du TBS (Tris Buffer Saline). Après une nuit, sous agitation et à température ambiante, la membrane a été lavée au TBS-Tween et l'anticorps AC1D4 dilué dans le tampon de saturation (C=1 µg/ml) a été ajouté. Ensuite, l'ensemble a été incubé, sous agitation, pendant une heure et demie à 37°C. Après lavage au TBS-Tween, l'addition d'un anticorps anti-IgG de souris couplé à la phosphatase alcaline a permis - après incubation pendant une heure à température ambiante et sous agitation - de révéler la présence de l'anticorps à étudier. Le complexe formé a été visualisé par ajout du substrat BCIP (sel de 5-bromo, 4-chloro, 3-indolyl phosphate toluidinium) contenant du chlorure de magnésium et du bleu de Thiazolyle dans une solution saline de tampon citrate CBS (citrate buffer saline).

Le peptide reconnu par l'anticorps AC1D4 est le peptide ayant la séquence :

Leu Gln Lys Arg Gly lle Val Glu Gln

Cette séquence est située dans la jonction de la chaîne A de l'insuline et du C-peptide.

### 2.4. Paramètres cinétiques

Les paramètres ont été déterminés par le système BIAcore™. Ce système mesure en temps réel la liaison d'un anticorps à son antigène. L'unité de mesure est le RU, et cette méthode permet la détermination de la constante de vitesse d'association (Kon), de la constante de vitesse de dissociation (Koff) et enfin le calcul de la constante (KA) de la liaison de l'anticorps à la proinsuline humaine. Les résultats de cette étude sont donnés dans le Tableau 1.

**TABLEAU I**

| **Anticorps Monoclonal** | **AC1D4** |
|---|---|
| Signal RU | 1305 |
| Koff (s-¹) | 1,65 10⁻⁵ |
| Kon (M⁻¹s⁻¹) | 1,16 10⁵ |
| KA (M⁻¹) | 7 10⁹ |

### 2.5. Liaison d'un anticorps monoclonal anti-C-peptide à la proinsuline humaine en présence et en absence de l'anticorps AC1D4.

Un anticorps monoclonal anti-C-peptide, le PEP001 et l'anticorps AC1D4 ont été testés par le système BlAcore™ pour leur capacité à se fixer simultanément sur la proinsuline couplée au dextran. L'anticorps monoclonal PEP001 est fourni par la société DAKO et reconnaît un épitope du C-peptide correspondant à la séquence suivante :

Pro Gly Ala Gly Ser Leu Gln Pro Leu Ala Leu Glu Gly Ser

x

Cet épitope est adjacent à l'épitope: reconnu par l'anticorps AC1D4 sur la proinsuline humaine.

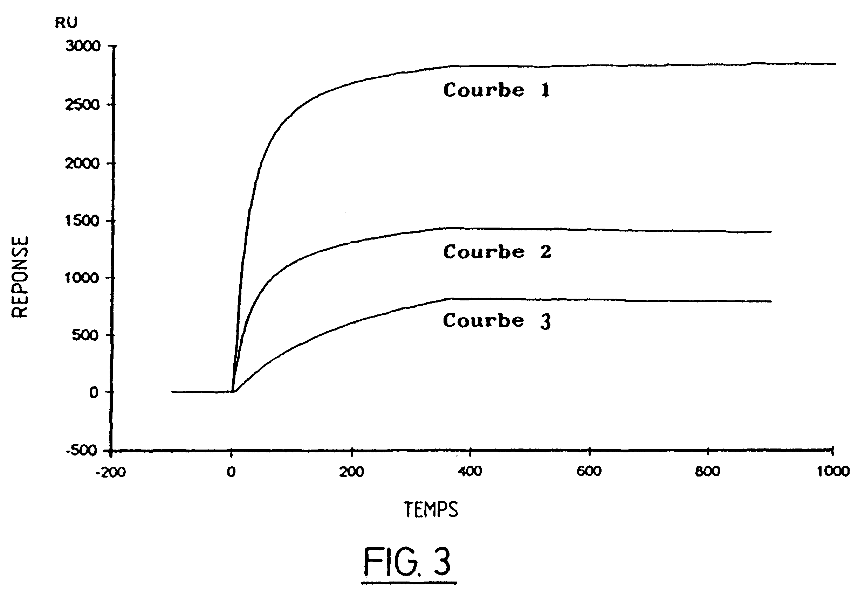

Pour cet essai, ont été utilisées des quantités croissantes de l'anticorps. AC1D4. Les résultats de cette étude sont donnés à la figure 3.
- La courbe 1 représente la liaison de l'anticorps PEP001 sur la proinsuline en absence de l'anticorps AC1D4.
- La courbe 2 représente la liaison de l'anticorps PEP001 sur la proinsuline. en présence de l'anticorps AC1D4 à la même concentration que l'anticorps PEP001. Dans ce cas on observe une inhibition de 50% de la liaison de l'anticorps PEP001 par l'anticorps AC1D4.
- La courbe 3 représente la liaison de l'anticorps PEP001 sur la proinsuline en présence de l'anticorps AC1D4 à une concentration 10 fois supérieure par rapport à la concentration de l'anticorps PEP001. Dans ce cas, on observe une inhibition de 70% de la liaison l'anticorps PEP001 par l'anticorps AC1D4.

Les exemples suivants illustrent l'invention et sont donnés à titre non limitatif.

### EXEMPLE 1 :

### Procédé de dosage spécifique du C-peptide dans le sérum : Dosage immunoenzymatique de type « sandwich ». Evaluation des conditions optimales.

Pour la réalisation de cet essai, on a utilisé un appareil automatique de type Access™. L'essai est réalisé avec 20 µl de sérum. Deux anticorps spécifiques de la proinsuline ont été utilisés :
- l'anticorps AC1D4 reconnaissant spécifiquement la proinsuline et la des-31,32-proinsuline et
- l'anticorps BC9B6 reconnaissant spécifiquement la proinsuline et la des-64,65-proinsuline.

Ce dernier anticorps reconnaît un épitope de la jonction BC correspondant à la séquence peptidique :

Thr Pro Lys Thr Arg Arg Glu Ala Glu Asp Leu Gln Val

Pour déterminer la concentration des anticorps AC1D4 et BC9B6 nécessaires pour éliminer les interférences dues à la proinsuline intacte, la des-31,32-proinsuline et la des-64,65-proinsuline, on a utilisé des concentrations croissantes de ces anticorps, seuls ou en mélange.

Comme phase solide, on a utilisé des billes de latex ferriques (Rhône Poulenc réf. MI-070/60) sur lesquelles est fixé par des liaisons covalentes l'anticorps monoclonal anti-C-peptide, PEP001.

Dans le tube de lecture sont introduits dans l'ordre :
1. 50 µl de billes revêtues d'anticorps monoclonal anti-C-peptide,
2. 20 µl de sérum ayant subi un traitement préalable au charbon actif et dans lequel on introduit des concentrations croissantes de proinsuline ou de
   des-31,32-proinsuline ou de des-64,65-proinsuline,
3. 80 µl de tampon pH=8 composé de Tris 0,02 M, NaCl 0,15 et des conservateurs,
   et
4. 50 µl de conjugué anticorps polyclonal de chèvre anti-C-peptide phosphatase alcaline dans un tampon pH=8 (constitué de Tris 0,1 M, de MgCl₂ 2mM, de ZnCl₂ 0,1 M, de NaCl 0,15M et additionné d'une protéine et des conservateurs) et contenant l'anticorps monoclonal AC1D4 et l'anticorps monoclonal BC986 à différentes concentrations : 0 µg/ml, 1 µg/ml, 5 µg/ml, 10µg/ml et 20 µg/ml.

Après 30 minutes d'incubation à 37°C, on lave trois fois avec un tampon pH=8 (composé de Tris 0,02 M, de NaCl 0,15 et des conservateurs) et on ajoute 200µl de substrat Lumi-Phos™ 530. On incube à nouveau pendant 5 minutes à 37°C et on mesure la luminescence générée par la réaction avec un luminomètre.

Les résultats de cette étude sont donnés dans les tableaux Il à IV. Les résultats sont exprimés en pourcentage de réaction croisée.

**TABLEAU II**

| AC1D4 | 0 µg/ml | 20 µg/ml |
|---|---|---|
| Proinsuline | 92 | 8 |
| Des-31,32-proinsuline | 108 | 16 |
| Des-64,65-proinsuline | 94 | 85 |

**TABLEAU III**

| BC9B6 | 0µg/ml | 20µg/ml |
|---|---|---|
| Proinsuline | 92 | 8 |
| Des-31,32-proinsuline | 108 | 115 |
| Des-64,65-proinsuline | 94 | 8 |

**TABLEAU IV**

| AC1D4+BC9B6 Q* _{AC1D4} = Q_{BC9B6} | 0µg/ml | 2µg/ml | 10µg/ml | 20µg/ml | 40µg/ml |
|---|---|---|---|---|---|
| Proinsuline | 92 | 23 | 4 | 2 | 1,8 |
| Des-31,32-proinsuline | 108 | 65 | 30 | 19 | 13 |
| Des-64,65-proinsuline | 94 | 48 | 18 | 10 | 7 |
| Q*_{AC1D4} : Quantité de AC1D4 | | | | | |

Les résultats du Tableau lV démontrent que lorsqu'on ajoute le mélange des anticorps AC1D4 et BC9B6 d'une concentration chacun de 20µg/ml, il est possible d'obtenir une réaction croisée avec la proinsuline inférieure à 5%, une réaction croisée avec la des-31,32-proinsuline inférieure à 15% et une réaction croisée avec la des-64,65-proinsuline inférieure à 10%.

### EXEMPLE 2 :

### Procédé de dosage spécifique du C-peptide dans les urines : Dosage immunoenzymatique type « sandwich »

Pour doser le C-peptide dans les urines, il est possible de procéder comme indiqué dans l'exemple 1, en diluant préalablement l'échantillon de l'urine au 1/20ème.

En ajoutant l'anticorps AC1D4 à une concentration de 30 µg/ml, et l'anticorps BC9B6 à une concentration de 5 µg/ml, il est possible d'obtenir une interférence due à la proinsuline inférieure à 5%, une interférence due à la des-31,32-proinsuline inférieure à 10%, et une interférence due à la des-64,65-proinsuline inférieure à 20%.

### EXEMPLE 3 :

### Procédé de dosage spécifique du C-peptide dans le sérum : Dosage radioimmunologique par compétition.

Le dosage repose sur la compétition entre une quantité fixe de tyr-C-peptide marqué à l'iode (I¹²⁵) et le C-peptide contenu dans les solutions étalons ou les échantillons à doser, vis à vis d'un nombre limité de sites anticorps anti-C-peptide.

Pour réaliser ce dosage, on utilise des tubes polystyrène revêtus avec un anticorps monoclonal anti-C-peptide PEP001 et on procède comme suit :
Dans chaque tube on introduit :
   - 100 µl d'une solution standard, contenant de 0,2 à 30 ng/ml de C-peptide en solution dans une solution tampon phosphate pH=6,8 additionné de protéines et contenant 0,1 % d'azoture de sodium, ou 100 µl de l'échantillon à doser,
      et
   - 50 µl d'une solution contenant de Tyr-C-peptide monoiodé dilué dans un tampon phosphate contenant 0,1% d'azoture de sodium et l'anticorps AC1D4 dirigé spécifiquement contre la jonction AC de la molécule de la proinsuline à 20 µg/ml.

On recouvre les tubes de Parafilm™. Après incubation pendant 2 heures à température ambiante et sous agitation horizontale, on élimine le milieu réactionnel par aspiration et on lave avec 2 ml d'une solution tampon pH=7,4 contenant de l'imidazole 0,05 M, de l'azide de sodium 0,0025% et de conservateurs. On élimine cette solution par aspiration et on répète deux fois encore l'opération du lavage.

Ensuite, on détermine la, radioactivité de chaque tube à l'aide d'un scintillateur gamma réglé sur la mesure de I¹²⁵. Le comptage est effectué pendant une minute.

L'évaluation de la quantité de C-peptide est effectuée par rapport à une gamme d'étalonnage. La limite de détection de cette méthode est de 0,15 ng/ml.

Pour évaluer la spécificité de ce dosage, deux sérums ont été surchargés avec de la proinsuline, de la des-31,32-proinsuline et de la des-64,65-proinsuline à différentes concentrations.

L'essai a été effectué en présence et en absence de l'anticorps AC1D4 (C=20 µg/ml). Les résultats de cet essai sont indiqués dans les tableaux V à VII.

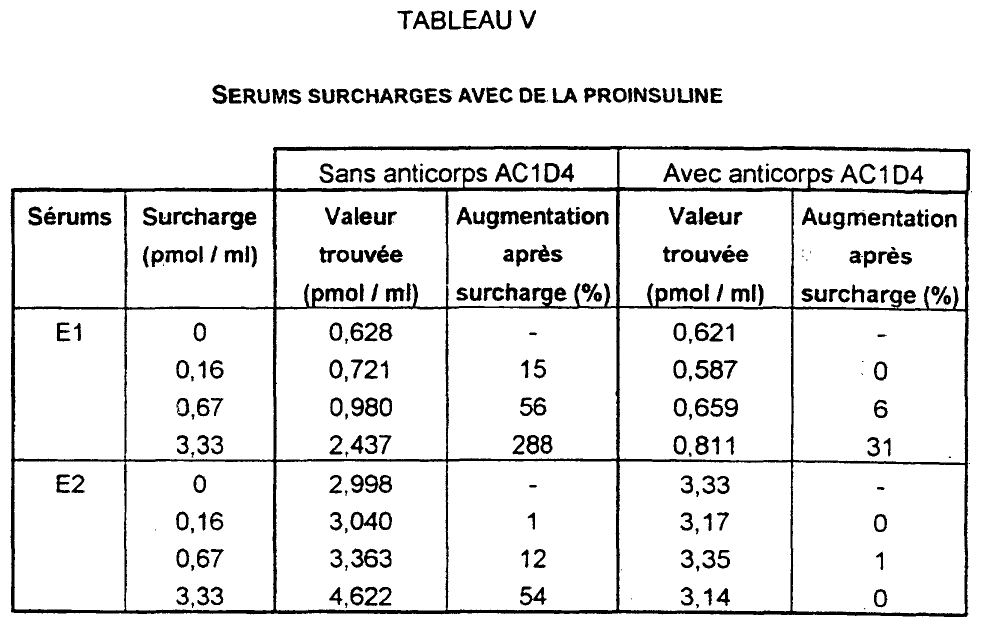

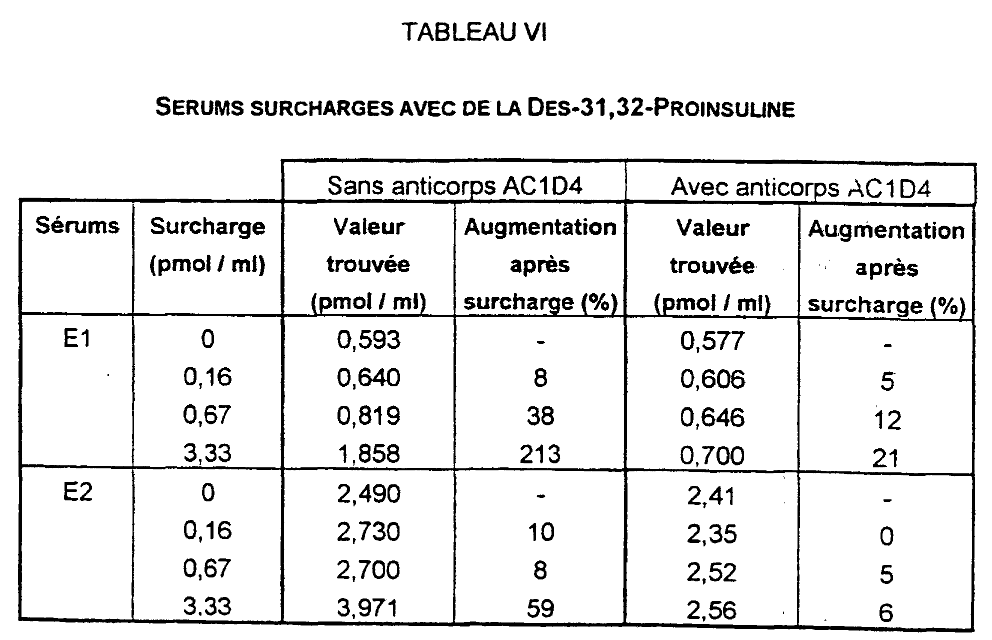

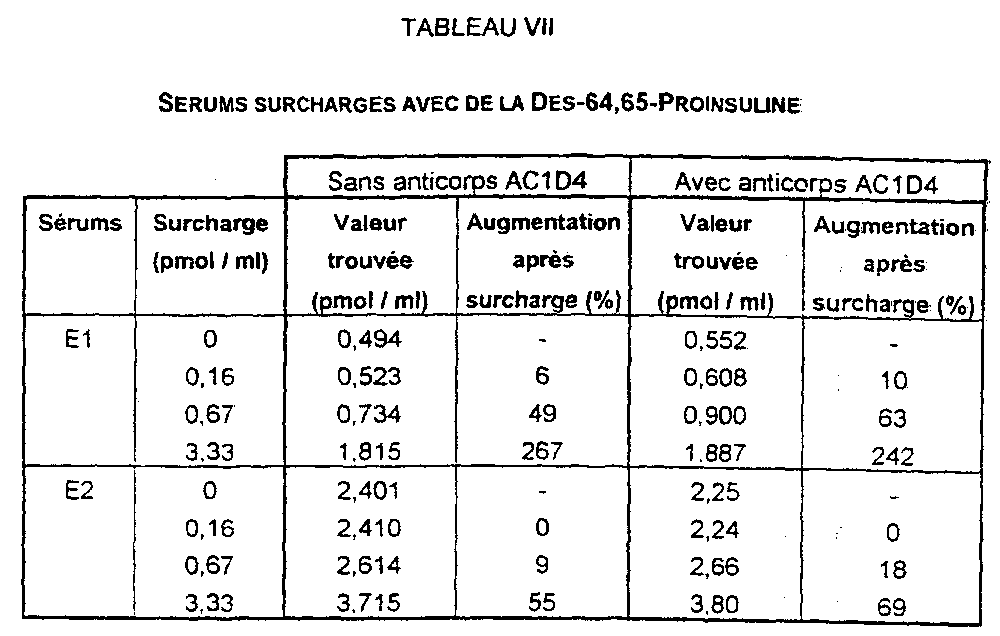

Les résultats de cette étude démontrent que la présence d'un anticorps monoclonal spécifique de la jonction AC de la proinsuline permet l'élimination de t'interférence due à la proinsuline, et à la des-31,32-proinsuline dans le dosage du C-peptide utilisant l'anticorps PEP001 dont l'épitope est proche de celui de l'AC1D4.

## Revendications

1. Procédé de dosage du C-peptide dans lequel un échantillon susceptible de contenir le C-peptide est mis en contact avec un ou plusieurs anticorps anti-C-peptide et
- soit un anticorps anti-proinsuline spécifique de la proinsuline intacte et de la des-31,32-proinsuline, reconnaissant un épitope soit adjacent, soit chevauchant, à l'épitope de l'anticorps anti-C-peptide gênant ainsi la fixation de l'anticorps anti-C-peptide sur la proinsuline intacte et la des-31,32-proinsuline,
- soit un anticorps anti-proinsuline spécifique de la proinsuline intacte et de la des-64,65-proinsuline, reconnaissant un épitope soit adjacent, soit chevauchant, à l'épitope de l'anticorps anti-C-peptide gênant ainsi la fixation de l'anticorps anti-C-peptide sur la proinsuline intacte et la des-64,65-proinsuline,
- soit un mélange dudit anticorps anti-proinsuline spécifique de la proinsuline intacte et de la des-31,32-proinsuline et dudit anticorps anti proinsuline spécifique de la proinsuline intacte et de la des-64,65-proinsuline.

2. Procédé de dosage du C-peptide, selon la revendication 1, dans lequel ledit anticorps anti-proinsuline spécifique de la proinsuline intacte et de la des-31,32-proinsuline, reconnaît un épitope situé dans la jonction AG de la proinsuline et la des-31,32-proinsuline, ledit épitope incluant la séquence peptidique :
Lys Arg.

3. Procédé de dosage du C-peptide, selon l'une quelconque des revendications précédentes, dans lequel ledit anticorps anti-proinsuline spécifique de la proinsuline intacte et de la des-31,32-proinsuline, reconnaît un épitope situé dans la jonction AC de la proinsuline et la des-31,32-proinsuline, ledit épitope incluant la séquence peptidique :
Leu Gln Lys Arg Gly lle Val Glu.

4. Procédé de dosage du C-peptide, selon l'une des revendications précédentes, dans lequel ledit anticorps anti-proinsuline spécifique de la proinsuline intacte et de la des-31,32-proinsuline, reconnaît un épitope situé dans la jonction AC de la proinsuline et la des-31,32-proinsuline, ledit épitope incluant la séquence peptidique :
Leu Gln Lys Arg Gly Ile Val Glu Gln.

5. Procédé de dosage du C-peptide, selon l'une des. revendications précédentes, dans lequel ledit anticorps anti-proinsuline. spécifique de la proinsuline intacte et de la des-64,65-proinsuline, reconnaît un épitope situé dans la jonction BC de la proinsuline et la des-64,65-proinsuline, ledit épitope incluant la séquence peptidique :
Arg Arg.

6. Procédé de dosage du C-peptide, selon l'une des revendications précédentes, dans lequel un échantillon- susceptible de contenir le C-peptide est mis en contact avec un anticorps anti-C-peptide et un anticorps anti-proinsuline spécifique de la proinsuline intacte et de la des-31,32-proinsuline reconnaissant un épitope soit adjacent, soit chevauchant, à l'épitope de l'anticorps anti-C-peptide gênant ainsi la fixation de l'anticorps anti-C-peptide sur la proinsuline intacte et la des-31,32-proinsuline.

7. Procédé de dosage du C-peptide , selon l'une des revendications 1 à 5, dans lequel un échantillon susceptible de contenir le C-peptide est mis en contact avec un anticorps anti-C-peptide et un anticorps anti-proinsuline spécifique de la proinsuline intacte et de la des-64,65-proinsuline reconnaissant un épitope soit adjacent, soit chevauchant, à l'épitope de l'anticorps anti-C-peptide gênant ainsi la fixation de l'anticorps anti-C-peptide sur la proinsuline intacte et la des-64,65-proinsuline.

8. Procédé de dosage du C-peptide, selon l'une quelconque des revendications précédentes, dans lequel au moins un desdits anticorps est un anticorps monoclonal.

9. Procédé de dosage du C-peptide, selon l'une quelconque des revendications précédentes, dans lequel l'échantillon est obtenu à partir de sérum, de plasma ou d'urines.

10. Procédé de dosage du C-peptide, selon l'une quelconque des revendications précédentes, de type « sandwich ».

11. Procédé de dosage du C-peptide, selon l'une quelconque des revendications 1 à 9, de type dosage radioimmunologique par compétition.

12. Anticorps spécifique de la des-31,32-proinsuline et de la proinsuline qui reconnaît la séquence peptidique :
Leu Gln Lys Arg Gly lle Val Glu Gln.

13. Hybridome déposé auprès de la Collection Nationale de Cultures de Microorganismes de l'Institut Pasteur, le 4 juin 1997, sous le numéro 1-1873.

14. Anticorps monoclonal spécifique de la des-31,32-proinsuline et de la proinsuline selon la revendication 12, qui est obtenu à partir de l'hybridome selon la revendication 13.

15. Utilisation d'anticorps monoclonaux qui reconnaissent un épitope situé dans la jonction AC de la proinsuline et la des-31,32-proinsuline incluant la séquence peptidique :
Lys Arg
pour la mise en oeuvre du procédé de dosage selon l'une des revendications 1 à 6 et 8 à 11.

16. Utilisation selon la revendication 15, d'anticorps. monoclonaux, qui reconnaissent un épitope incluant la séquence choisie parmi:
la séquence Leu Gln Lys Arg Gly Ile Val Glu
et la séquence Leu Gln Lys Arg Gly Ile Val Glu Gln.

17. Utilisation d'anticorps monoclonaux qui reconnaissent un épitope situé dans la jonction BC de la proinsuline et la des-64,65-proinsuline incluant la séquence peptidique:
Arg Arg
pour la mise en oeuvre du procédé de dosage selon l'une des revendications 1 à 5 et 7 à 11.

18. Utilisation selon la revendication 17, d'anticorps monoclonaux, qui reconnaissent un épitope correspondant à une séquence peptidique choisie parmi :
la séquence Phe Tyr Thr Pro Lys Thr Arg Arg Glu Ala Glu Asp Leu Gln Val,
la séquence Thr Pro Lys Thr Arg Arg Glu Ala Glu Asp Leu Gln Val,
et la séquence Phe Tyr Thr Pro Lys Thr Arg Arg Glu Ala Glu Asp Leu.

19. Utilisation d'une trousse contenant un anticorps anti-C-peptide et un anticorps spécifique de la des-31,32-proinsuline et de la proinsuline et/ou un anticorps spécifique de la des-64,65-proinsuline et de la proinsuline, pour le dosage du C-peptide.

## Claims

1. Process for titrating C-peptide, wherein a sample likely to contain the C-peptide is brought into contact with one or more anti-C-pepride antibodies and
- either an anti-proinsulin antibody specific for intact proinsulin and des-31,32-proinsulin, recognising an epitope either adjacent to or overlapping with the epitope of the anti-C-peptide antibody, thus preventing the anti-C-peptide from binding to the intact proinsulin and the des-31,32-proinsulin,
- or an anti-proinsulin antibody specific for intact proinsulin and des-64,65-proinsulin, recognising an epitope either adjacent to or overlapping with the epitope of the anti-C-peptide antibody, thus preventing the anti-C-peptide from binding to the intact proinsulin and the des-64,65-proinsulin,
- or a mixture of said anti-proinsulin antibody specific for intact proinsulin and des-31,32-proinsulin and of said anti-proinsulin antibody specific for intact proinsulin and des-64,65-proinsulin.

2. Process for titrating C-peptide according to claim 1, wherein said anti-proinsulin antibody specific for intact proinsulin and des-31,32-proinsulin recognises an epitope located at the AC junction of the proinsulin and des-31,32-proinsulin, said epitope including the peptide sequence:
Lys Arg.

3. Process for titrating C-peptide according to any one of the preceding claims) wherein said anti-proinsulin antibody specific for intact proinsulin and des-31,32-proinsulin recognises an epitope located at the AC junction of proinsulin and des-31,32-proinsulin, said epitope including the peptide sequence:
Leu Gln Lys Arg Gly Ile Val Glu.

4. Process for titrating C-peptide according to one of the preceding claims, wherein said anti-proinsulin antibody specific for intact proinsulin and des-31,32-proinsulin recognises an epitope located at the AC junction of proinsulin an d des-31,32-proinsulin, said epitope including the peptide sequence:
Leu Gln Lys Arg Gly Ile Val Glu Gln.

5. Process for titrating C-peptide according to one of the preceding claims, wherein said and-proinsulin antibody specific for intact proinsulin and des-64,65-proinsulin recognises an epitope located at the BC junction of proinsulin and des-64,65-proinsulin, said epitope including the peptide sequence:
Arg Arg.

6. Process for titrating C-peptide according to any one of the preceding claims, wherein a sample likely to contain the C-peptide is brought into contact with an anti-C-peptide antibody and an anti-proinsulin antibody specific for intact proinsulin and des-31,32-proinsulin recognising an epitope which is either adjacent to or overlapping with the epitope of the anti-C-peptide antibody, thus preventing the anti-C-peptide antibody from binding to the intact proinsulin and the des-31,32-proinsulin.

7. Process for titrating C-peptide according to one of claims 1 to 5, wherein a sample likely to contain the C-peptide is brought into contact with an and-C-pepdde antibody and an anti-proinsulin antibody specific for intact proinsulin and des-64,65-proinsulin recognising an epitope which is either adjacent to or overlapping with the epitope of the anti-C-peptide antibody, thus preventing the anti-C-peptide antibody from binding to the intact proinsulin and the des-64,65-proinsulin.

8. Process for titrating C-peptide according to any one of the preceding claims, wherein at least one of said antibodies is a monoclonal antibody.

9. Process for titrating C-peptide according to any one of the preceding claims, wherein the sample is obtained from serum, plasma or urine.

10. Process for titrating C-peptide according to any one of the preceding claims, of the "sandwich" type.

11. Process for titrating C-peptide according to one of claims 1 to 9 of the competitive radioimmunological assay type.

12. Antibody specific for des-31,32-proinsulin and proinsulin which recognises the peptide sequence:
Leu Gln Lys Arg Gly Ile Val Glu Gln.

13. Hybridoma deposited at the National Collection for Microorganism Cultures at the Institut Pasteur on 4 June 1997 under the number I-1873.

14. Monoclonal antibody specific for des-31,32-proinsulin and proinsulin according to claim 12, which is obtained from the hybridoma according to claim 13.

15. Use of monoclonal antibodies which recognise an epitope located at the AC junction of proinsulin and des-31,32-proinsulin including the peptide sequence:
Lys Arg
for carrying out the titration process according to any one of claims 1 to 6 and 8 to 11.

16. Use according to claim 15 of monoclonal antibodies which recognise an epitope including the sequence selected from among:
the sequence Leu Gln Lys Arg Gly Ile Val Glu
and the sequence Leu Gln Lys Arg Gly Ile Val Glu Gln.

17. Use of monoclonal antibodies which recognise an epitope located at junction BC of the proinsulin and the des-64,65-proinsulin including the peptide sequence:
Arg Arg
for carrying out the titration process according to one of claims 1 to 5 and 7 to 11.

18. Use according to claim 17 of monoclonal antibodies which recognise an epitope corresponding to a peptide sequence selected from among:
the sequence Phe Tyr Thr Pro Lys Thr Arg Arg Glu Ala Glu Asp Leu Gln Val,
the sequence Thr Pro Lys Thr Arg Arg Glu Ala Glu Asp Leu GIn Val,
and the sequence Phe Tyr Thr Pro Lys Thr Arg Arg Glu Ala Glu Asp Leu.

19. Use of a kit containing an anti-C-peptide antibody and an antibody specific for des-31,32-proinsulin and proinsulin and/or an antibody specific for des-64,65-proinsulin and proinsulin, for titrating C-peptide.

## Patentansprüche

1. Verfahren zum Dosieren von C-Peptid, in dem eine Probe, die zum Enthalten des C-Peptids geeignet ist, in Kontakt mit einem oder mehreren Anti-C-Peptid-Antikörpern gebracht wird und
- entweder einem Antiproinsulin-Antikörper, der für intaktes Proinsulin und Des-31,32-Proinsulin spezifisch ist und entweder ein zu dem Epitop des Anti-C-Peptid-Antikörpers benachbartes oder dieses überlappendes Epitop erkennt und somit ein Binden des Anti-C-Peptid-Antikörpers an dem intakten Proinsulin. und dem Des-31,32-Proinsulin verhindert,
- oder einem Antiproinsulin-Antikörper, der für intakes Proinsulin und Des-64,65-Proinsulin spezifisch ist und entweder ein zu dem Epitop des Anti-C-Peptid-Antikörpers benachbartes oder überlappendes Epitop erkennt und somit das Binden des Anti-C-Peptid-Antikörpers an dem intakten Proinsulin und dem Des-64,65-Proinsulin verhindert,
- oder einem Gemisch dieses Antiproinsulin-Antikörpers, der spezifisch für das intakte Proinsulin und das Des-31,32-Proinsulin ist und des Antiproinsulin-Antikörpers, der spezifisch für das intakte Proinsulin und das Des-64,65-Proinsulin ist.

2. Verfahren zum Dosieren von C-Peptidnach Anspruch 1, in dem der Antiproinsulin-Antikörper, der spezifisch für das intakte Proinsulin und das Des-31,32-Proinsulin ist, ein Epitop, das in der Verbindung AC des Proinsulins und des Des-31,32-Proinsulins liegt, erkennt, wobei dieses Epitop die folgende Peptidsequenz aufweist:
Lys Arg.

3. Verfahren zum Dosieren von C-Peptidnach einem der vorangehenden Ansprüche, in dem der Antiproinsulin-Antikörper, der spezifisch für das intakte Proinsulin und das Des-31,32-Proinsulin ist, ein Epitop erkennt, das in der Verbindung AC des Proinsulins und des Des-31,32-Proinsulins liegt, wobei das Epitop die folgende Peptidsequenz aufweist:
Leu Gln Lys Arg Gly Ile Val Glu.

4. Verfahren zum Dosieren von C-Peptid nach einem der vorangehenden Ansprüche, in dem der Antiproinsulin-Antikörper, der spezifisch für das intakte. Proinsulin und das Des-31, 32-Proinsulin ist, ein Epitop erkennt, das in der Verknüpfung AC des Proinsulins und des Des-31,32-Proinsulins liegt, erkennt, wobei dieses Epitop die folgende Peptidsequenz aufweist:
Leu Gln Lys Arg Gly Ile Val Glu Gln.

5. Verfahren zum Dosieren von C-Peptid nach einem der vorangehenden Ansprüche, in dem der Antiproinsulin-Antikörper, der spezifisch für das intakte Proinsulin und das Des-64, 65-Proinsulin ist, ein Epitop, das in der Verbindung BC des Proinsulins und des Des-64,65-Proinsulins liegt, erkennt, wobei dieses Epitop die folgende Peptidsequenz aufweist:
Arg Arg.

6. Verfahren zum Dosieren von C-Peptid nach einem der vorangehenden Ansprüche, in dem eine Probe, die zum Enthalten des C-Peptid s geeignet ist, in Kontakt gebracht wird mit einem Anti-C-Peptid -Antikörper und einem Antiproinsulin-Antikörper, der spezifisch für das intakte Proinsulin und das Des-31,32-Proinsulin ist und entweder ein zu dem Epitop des Anti-C-Peptid -Antikörpers benachbartes oder dieses überlappendes Epitop erkennt und somit die Bindung des Anti-C-Peptid -Antikörpers an dem intakten Proinsulin und dem Des-31,32-Proinsulin verhindert.

7. Verfahren zum Dosieren von C-Peptid nach einem der Ansprüche 1 bis 5, in dem eine Probe, die zum Enthalten des C-Peptid s geeignet ist, in Kontakt gebracht wird mit einem Anti-C-Peptid -Antikörper und einem Antiproinsulin-Antikörper, der spezifisch für das intakte Proinsulin und das Des-64,55-Proinsulin ist und entweder ein zu dem Epitop des Anti-C-Peptid -Antikörpers benachbartes oder dieses überlappendes Epitop erkennt und somit die Bindung des Anti-C-Peptid -Antikörpers an dem intakten Proinsulin und dem Des-64,65-Proinsulin verhindert.

8. Verfahren zum Dosieren von C-Peptid nach einem der vorangehenden Ansprüche, in dem mindestens einer, der Antikörper ein monoklonaler Antikörper ist.

9. Verfahren zum Dosieren von C-Peptid nach einem der vorangehenden Ansprüche, in dem die Probe aus Serum, Plasma oder Urin gewonnen wurde.

10. Verfahren zum Dosieren von C-Peptid, nach einem der vorangehenden Ansprüche vom Typ "Sandwich".

11. Verfahren zum Dosieren von C-Peptid, nach einem der Ansprüche 1 bis 9, vom Typ kompetitiver radioimmunologischer Dosierung.

12. Antikörper, spezifisch für das Des-31,32-Proinsulin und Proinsulin, der die folgende Peptidsequenz erkennt:
Leu Gln Lys Arg Gly Ile Val Glu Gln.

13. Hybridom, hinterlegt am 4. Juni 1997 bei der Collection Nationale de Cultures de Microorganismes de l'Institut Pasteur unter der Nummer I-1873.

14. Monoklonaler Antikörper, spezifisch für das Des-31,32-Proinsulin und das Proinsulin nach Anspruch 12, der durch das Hybridom nach Anspruch 13 erhalten wird.

15. Verwendung monoklonaler Antikörper, die ein Epitop erkennen, das in der Verknüpfung AC des Proinsilins und des Des-31,32-Proinsulins liegt und das folgende Peptidsequenz aufweist:
Lys Arg
zur Durchrührung des Dosierungsverfahren nach einem der Ansprüche 1 bis 6 und 8 bis 11.

16. Verwendung monoklonaler Antikörper nach Anspruch 15, die ein Epitop erkennen, das die Sequenz aufweist, die ausgewählt ist aus:
der Sequenz Leu Gln Lys Arg Gly Ile Val Glu
und der Sequenz Leu Gln Lys Arg Gly Ile Val Glu Gln.

17. Verwendung monoklonaler Antikörper, die ein Epitop erkennen, das in der Verknüpfung BC des Proinsulins und des Des-64 , 65-Proinsulins gelegen ist und das die folgende Peptidsequenz aufweist:
Arg Arg
zur Durchführung eines Verfahren zum Dosieren nach einem der Ansprüche 1 bis 5 und 7 bis 11.

18. Verwendung monoklonaler Antikörper nach Anspruch 17, die ein Epitop erkennen, das einer Peptidsequenz entspricht, die ausgewählt ist aus:
| | |
|---|---|
| der Sequenz | Phe Tyr Thr Pro Lys Thr Arg Arg Glu Ala Glu Asp Leu Gln Val , |
| der Sequenz | Thr Pro Lys Thr Arg Arg Glu Ala Glu Asp Leu Gln Val, |
| und der Sequenz | Phe Tyr Thr Pro Lys Thr Arg Arg Glu Ala Glu Asp Leu. |

19. Verwendung eines Behältnisses, enthaltend einen Anti-C-Peptid -Antikörper und einen Antikörper, der spezifisch für das Des-31,32-Proinsulin und das Proinsulin ist und/oder einen Antikörper, der spezifisch für das Des-64,65-Proinsulin und Proinsulin ist, zum Dosieren von C-Peptid en.
